Europäisches Patentamt

European Patent Office

Office européen des brevets

(19)

(11) Publication number: **0 118 306**
**B1**

(12) **EUROPEAN PATENT SPECIFICATION**

(45) Date of publication of patent specification: **25.05.88**

(51) Int. Cl.⁴: **A 61 N 1/362**

(21) Application number: **84301447.3**

(22) Date of filing: **05.03.84**

(54) **An atrial synchronized pacemaker having an adaptive atrial refractory period.**

(30) Priority: **04.03.83 US 471977**

(43) Date of publication of application:
**12.09.84 Bulletin 84/37**

(45) Publication of the grant of the patent:
**25.05.88 Bulletin 88/21**

(84) Designated Contracting States:
**DE FR GB IT NL SE**

(56) References cited:
**EP-A-0 039 269**
**EP-A-0 050 038**
**WO-A-83/01389**
**DE-A-3 217 190**
**FR-A-2 481 603**
**US-A-4 365 639**

(73) Proprietor: **MEDTRONIC, INC.**
**3055 Old Highway Eight P.O. Box 1453**
**Minneapolis Minnesota 55440 (US)**

(72) Inventor: **Citron, Paul**
**2712 Rice Creek Terrace**
**New Brighton, MN 55112 (US)**
Inventor: **Duffin, Edwing G.**
**201 17th Avenue SW**
**New Brighton, MN 55112 (US)**

(74) Representative: **Tomlinson, Kerry John et al**
**Frank B. Dehn & Co. European Patent Attorneys**
**Imperial House 15-19 Kingsway**
**London WC2B 6UZ (GB)**

## Description

### Background of the invention

Field of the invention—This invention relates generally to the field of heart pacemakers, and more specifically to improvements in atrial synchronized pacemakers.

Background of the prior art—The body's demand for oxygenated blood is reflected by the rate at which the sinus node of the heart beats. The electrical signal generated by the sinus node causes the atria or upper chambers of the heart to contract, forcing blood into the lower chambers or ventricles of the heart. After a brief delay, the lower chambers of the heart contract forcing the blood throughout the body. Normally, the ventricles contract approximately 100—200 milliseconds after the atria contract. This delay period is generated by a structure known as the A—V node, which receives a signal from the atria and relays it to the ventricles.

Pacemakers mimic the natural electrical system of the heart and provide therapeutic electrical stimulation pulses to the heart in response to cardiac conduction disturbances detected by the pacemaker.

The pacers described throughout this Specification are identified by the three-letter code adopted by the Intersociety Commission for Heart Disease Resources (ICHD). In this three-letter code, the first letter indicates the chamber paced, the second letter indicates the chamber of the heart which is sensed by the pacemaker and the third letter indicates the mode of response of the pacemaker. Details of this code may be found in the *American Journal of Cardiology*, 34 (487 1974).

The earliest form of pacer operated in the VOO mode. Pacers of this type pace the heart asynchronously with respect to the natural activity of the heart. An example of an early asynchronous pacemaker is known from U.S. Patent No. 3,057,356 issued to W. Greatbatch.

The later developed, demand or VVI mode pacemaker senses the ventricular contraction of the heart and provides stimulation to the ventricles only in the absence of a naturally occurring contraction of the ventricles. Such demand pacemakers are known from U.S. Patent No. 3,478,746 to W. Greatbatch.

The bifocal or DVI mode pacemaker provides sequential stimulation of the atrium and the ventricle if naturally occurring cardiac activity is not detected in the ventricle. This form of pacemaker is known from U.S. Patent No. 3,595,242 to B. Berkovits.

In each of the preceding pacemakers, electrical activity in the atrium is not sensed by the pacemaker. Consequently, these pacemakers do not utilize the information present in the atrial rate to govern the operation of the pacemaker.

In contrast, the P-synchronous or VAT mode pacemaker does sense electrical activity in the atrium. This form of pacer provides a stimulus to the ventricle to ensure that the ventricles depolarize in sequence with, and in synchrony with the activity of the atrium. Such VAT mode pacemakers are known from U.S. Patent No. 3,253,596 to J. W. Keller.

Another form of atrial synchronized pacemakers is the atrial synchronized, ventrical inhibited pacer or VDD mode pacer described in U.S. Patent No. 4,059,116 to J. M. Adams.

More recently the DDD mode pacemaker known from U.S. Patent No. 4,312,355 to H. Funke, has been introduced which also operates in an atrial tracking mode.

In the atrial tracking mode, the signal from the atrium is detected and utilized to time the generation of a ventricular pacing stimulus. In this fashion the atrial tracking pacer mimics the operation of the heart's A—V node, described previously.

An early form of the atrial tracking pacemaker is the VAT mode pacer known from U.S. Patent No. 3,253,596. This pacer tracks the naturally occurring atrial rate between a lower rate of typically 60 bpm to an upper rate of, for example, 120 bpm. Above the 120 bpm rate, the atrial refractory period built into the pacemaker would cause the pacemaker to pace the heart at one-half of the detected atrial rate. The blocking effect resulting from the atrial refractory period would cause the ventricular rate to suddenly drop from a relatively fast rate near 120 bpm to a relatively slow rate in the presence of an atrial rate above 120 bpm. Precipitous rate drops are uncomfortable for many patients and may comprise hemodynamic performance.

This problem was addressed by the Adams pacer which provided an upper rate stabilization circuit which would cause the ventricular stimulation rate to approach an upper limit and remain there for detected atrial rates above the upper rate limit defined by the pacemaker.

For example, in the early Keller pacemaker, if the atrial rate rose from a rate of 1150 bpm to 125 bpm, the ventricular rate would incrase from a rate of 115 bpm to 120 bpm and then drop precipitously to approximately 62 bpm as the atrial rate passed through 120 bpm. In contrast, the Adams pacemaker would track the atrial rate from 115 bpm up to 120 bpm and then stabilize at approximately 120 bpm as the atrial rate rose to 125 bpm.

This discussion has addressed the upper rate characteristics of atrial tracking pacemakers when the atrial rate is determined by the sinus node of the heart.

In the preceding description of the electrophysiology of the heart, the direction of conduction was assumed to be from the atrium to the ventricle. It appears, however, that in many patients there exists a retrograde conduction path from the ventricle into the atrium. This conductive path does not normally affect the operation of a pacemaker. However, it is possible for an atrial tracking pacemaker to detect this retrograde P-wave activity and synchronize upon it. This may result in the stimulation of the ventricles at a high rate and constitutes a pacemaker-mediated tachycardia which is undesirable.

DE—A—3217190 discloses a pacer which detects pacemaker-mediated tachycardia by sensing two ventricular contractions without an intervening atrial contraction and then stops the tachycardia by setting the atrial refractory period to 400 ms.

According to the invention there is provided a pacer for delivering stimulation pulses to the ventricle of a patient's heart in response to detected depolarizations of the atria of the patient's heart, comprising:

an atrial terminal means for coupling said pacer to the atrium of the patient's heart;

a ventricular terminal means for coupling said pacer to a ventricle of the patient's heart;

atrial sense amplifier means coupled to said atrial terminal means for generating an atrial sense signal in response to the detection of electrical activity in the atria;

A—V delay timer means coupled to said atrial sense amplifier means for generating an A—V delay period for generating a ventricular pace signal upon the expiration of said A—V delay period, said A—V delay period being initiated by an atrial sense signal;

ventricular pulse generator means coupled to said ventricular terminal means and said A—V delay timer means for producing a stimulation pulse in response to said ventricular pace signal;

an atrial refractory timer means arranged to prevent the initiation of said A—V delay timer during an atrial refractory period; characterised by

detection means responsive to said atrial sense signal for detecting retrograde activated P-waves, said detection means comprising a V—A time measuring means arranged to measure the time between the occurrence of a ventricular stimulating pulse and generation of an atrial sense signal and being coupled to said atrial refractory timer means for selecting said atrial refractory period to prevent retrograde activated P-waves from initiating a ventricular stimulation pulse.

The present invention is directed to a pacer which detects the existence of retrograde P-wave activity and which extends the atrial refractory period or sets the atrial refractory period of the pacemaker to prevent the synchronization upon retrograde P-activity, thus preventing the maintenance of the pacemaker-mediated tachycardia.

Preferably, the invention provides a pacer further comprising:

an upper rate limit timer means coupled to said ventricular pulse generator means for defining an upper rate limit interval for preventing the generation of a ventricular stimulating pulse during said upper rate limit interval, and for generating a ventricular pace signal at the expiration of said upper rate limit interval if a ventricular pace signal is generated during such upper rate limit period, said upper rate limit timer being initiated by the delivery of a ventricular stimulation pulse;

said atrial refractory timer means being responsively coupled to said A—V delay timer means for preventing the initiation of said A—V delay timer until the expiration of an atrial refractory period, said atrial refractory period extending for a first predetermined time period after the occurrence of a ventricular stimulation pulse;

said A—V time measuring means being responsively coupled to said A—V delay timer means and said upper rate limit timer for measuring V—A time intervals, said V—A time interval extending from the occurrence of a ventricular stimulation pulse to the subsequent initiation of said A—V delay timer means; and including

atrial refractory control means coupled to said V—A measurement timer means and coupled to said atrial refractory time means for extending said first predetermined time period when said V—A time intervals are both short and invariant.

The invention also provides a pacer for delivering stimulating pulses to the ventricle and atria of a patient's heart in response to the presence or absence of the detection of electrical activity in the atria and ventricles of the patient's heart, comprising:

atrial terminal means for coupling said pacer to the atrium of the patient's heart;

ventricular terminal means for coupling said pacer to a ventricle of the patient's heart;

atrial sense amplifier means coupled to said atrial terminal means for generating an atrial sense signal in response to the detection of electrical activity in the atria;

ventricular sense amplifier means coupled to said ventricular terminal means for generating a ventricular sense signal in response to the detection of electrical activity in the ventricle;

A—V delay timer means responsively coupled to said atrial sense amplifier means and said ventricular sense amplifier means for generating an A—V delay period, said A—V delay period being initiated by an atrial sense signal and said A—V delay timer being reset by said ventricular sense signal, and for generating a ventricular pace signal upon the expiration of said A—V delay period;

ventricular pulse generator means coupled to said ventricular terminal means for generating a ventricular stimulation signal in response to a ventricular pace signal;

an atrial pulse generator means coupled to said atrial terminal means for generating an atrial stimulation signal in response to an atrial pace signal;

lower rate timer means coupled to said ventricular pulse generator means and said atrial pulse generator means and said ventricular sense amplifier means for defining lower V—V, and V—A rate escape intervals and for producing an atrial pace signal at the expiration of said V—A time interval and for producing a ventricular pace signal at the expiration of said V—V time interval, said lower rate timer being initiated by said ventricular sense signal or said ventricular pace signal;

an atrial refractory timer means responsively coupled to said A—V delay timer means, and to said atrial sense amplifier means, and to said ventricular pulse generator for preventing the initiation of said A—V delay timer until the expiration of an atrial refractory period, said atrial refractory period extending for a first predetermined time period after the occurrence of a ventricular stimulation pulse;

a ventricular refractory timer means coupled to said lower rate timer means and said ventricular sense amplifier means for defining a ventricular refractory period for preventing the initiation of said lower rate timer means during said ventricular refractory period, said ventricular refractory period being initiated on the occurrence of a ventricular stimulation pulse, and said ventricular refractory period terminating a first predetermined time after initiation;

a V—A measurement timer means responsively coupled to said A—V timer and to said ventricular pulse generator for measuring the time period from the occurrence of a ventricular stimulation pulse to the subsequent initiation of said A—V delay timer;

atrial refractory control means coupled to said V—A timer means and coupled to said atrial refractory timer means for extending said first predetermined time period when said V—A time periods are both short and invariant; and

an upper rate limit timer means coupled to said ventricular pulse generator means for defining an upper rate limit interval for preventing the generation of a ventricular stimulating pulse during said upper rate limit interval, and for generating a ventricular pace signal at the expiration of said upper rate limit interval if a ventricular pace signal is generated during such upper rate limit period, said upper rate limit timer being initiated by the occurrence of a ventricular event.

Certain embodiments of the invention will now be described by way of example and with reference to the accompanying drawings.

Description of the drawings

Figure 1 represents a functional block diagram of a simple VAT mode pacer incorporating the invention;

Figure 2 is a schematic timing diagram illustrating the upper rate behavior of the pacer of Figure 1 at high sinus rates;

Figure 3 is a schematic timing diagram illustrating the behavior of the pacer of Figure 1 in the presence of retrograde activated atrial activity;

Figure 4 is a diagram illustrating the detection procedure used in the invention; and

Figure 5 is a functional block diagram of a DDD mode pacer incorporating the invention.

Detailed description of preferred embodiments

As previously described, the present invention may be utilized in any atrial tracking pacemaker. In the subsequent description, the invention will be described within the context of a VAT or atrial synchronized pacer. First, the general operation of such a VAT mode pacemaker will be discussed and subsequently the operation of such a pacemaker at high sinus (atrial) rates will be described. Next the operation of the pacemaker in response to the retrograde conduction of P-waves will be described illustrating the operation of the invention.

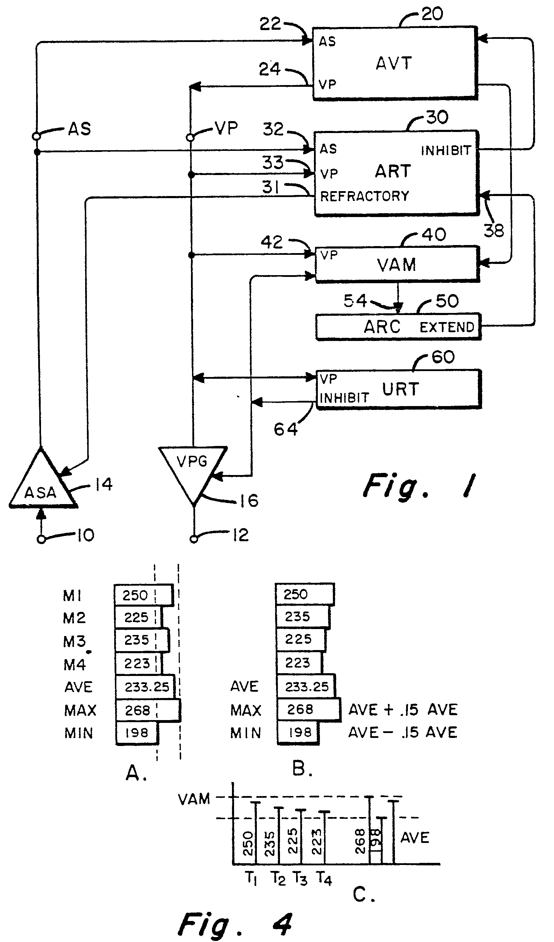

Figure 1 shows a VAT mode pacemaker which includes the present invention. The pacer is coupled to the atrium through an atrial terminal 10 and a ventricular terminal 12.

The atrial sense amplifier 14 (ASA) is coupled to the atrial terminal and generates an atrial sense signal (AS) in response to detected electrical activity within the atrium. This atrial sense signal is supplied through terminal 22 to an A—V delay timer 20 (AVT). The AVT 20 times out a programmed A—V delay time period and generates a ventricular pace signal (VP) at the expiration of the programmed A—V delay interval. This VP signal is supplied through terminal 24 to the ventricular pulse generator 16 (VPG). If the VPG is enabled, a ventricular pace signal supplied to the ventricular pulse generator will result in the generation of a ventricular pacing stimulus at ventricular terminal 12. These circuit elements cooperate to track the sensed atrial rate on a 1:1 basis to produce ventricular pacing stimuli in response to the detected atrial activity.

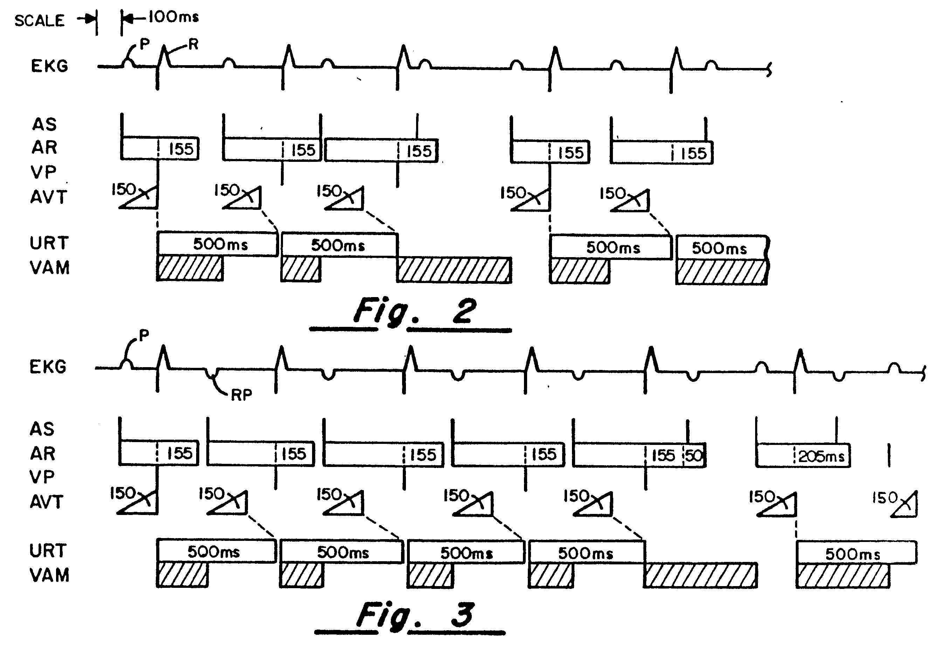

There is also shown an upper rate limit timer 60 (URT) which is involved in the upper rate stabilization characteristics of this pacemaker. The upper rate limit timer times out a programmed upper rate limit time period. This timer is initiated by the generation of a ventricular stimulation pulse. During the upper rate limit time interval, an inhibit signal is supplied through terminal 64 to the ventricular pulse generator 16, which prevents the generation of ventricular spacing stimuli during the upper rate limit time period. The interaction between this circuitry and the sensed atrial activity of the heart is depicted and described in connection with Figure 2 and Figure 3.

There is also shown an atrial refractory timer 30 (ART). The ART times out an atrial refractory period which prevents electrical activity present in the atrium from affecting the pacemaker's operation during the atrial refractory period. This objective may be achieved by the application of a control signal through terminal 31 of the ART which is applied to the atrial sense amplifier. This signal may disable the atrial sense amplifier or may be utilized to inhibit the generation of an atrial sense signal during the atrial refractory period. The atrial refractory period is initiated by the application of an atrial sense signal through terminal 32 of the ART. The atrial refractory period of the pacer shown normally ends 155 milliseconds after a VP signal is communicated to the ART through terminal 33.

In Figure 1 there is also shown a ventricular-atrial timer 40 (VAM) which measures the time interval from the generation of a ventricular stimulus to the subsequent reinitiation of the A—V delay timer 20. The VAM 40 provides beat-to-beat measurements of the V—A time interval, which is supplied to an atrial refractory control circuit 50 (ARC) through terminal 54. The ARC 50 additionally receives information through terminal 42 from the URT 60 whenever a VP signal is generated by the URT.

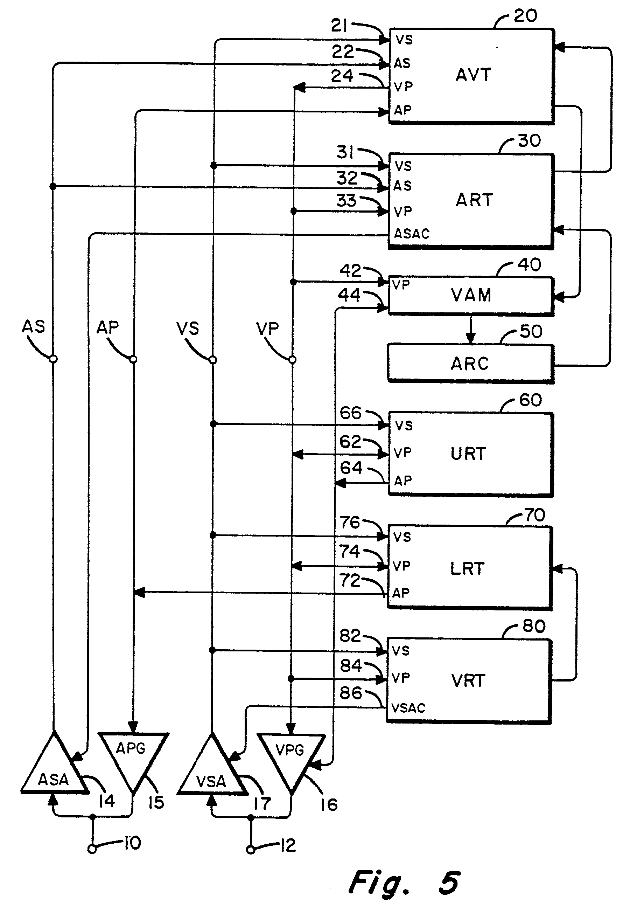

If the measured V—A time intervals are invariant within a small tolerance and the ventricular pace signals are occurring at the upper rate limit, then the ARC 50 will generate a control signal supplied to the ART 30 through terminal 38 to extend the refractory interval. As will be shown in connection with Figures 3 and 4, these criteria indicate that the pacemaker is participating in or mediating a tachycardia and the extension of the atrial refractory period will prevent the pacemaker from triggering on the retrograde P-wave activity.

Turning to Figure 2 there is shown a schematic timing diagram illustrating the operation of the pacemaker of Figure 1 at high atrial rates. In this example, the atrial activity is generated by the normal conduction of electrical activity from the atrium to the ventricle.

In the figure, atrial sense events detected by the ASA 14 are shown as vertical marks under the schematic P-wave shown on the EKG trace of the diagram. The duration of the atrial refractory period set by the ART 30 is shown as a block extending from the atrial sensed event and terminating 155 milliseconds after the occurrence of the ventricular paced event. The ventricular pace signal is shown under the pacing artifact adjacent the stimulated R-wave in the EKG. The time peroid associated with the A—V delay timer 20 is shown by the triangular figures on the diagram. This depicts the incrementing of a counter from zero to a programmed value shown on the figure as 150 milliseconds.

The time period associated with the upper rate timer URT 60 is shown by the blocks on the diagram adjacent the URT legend. The ventricular-atrial time periods measured by the pacemaker through the operation of VAM 40 are shown after the VAM legend on the diagram.

In operation, the first P-wave shown in the schematic EKG trace is detected by the atrial sense amplifier 14 giving rise to an atrial sense signal. This atrial sense signal starts the atrial refractory period and the A—V delay timer. The A—V delay timer times out generating a ventricular pace signal shown in the figure 150 milliseconds after the detection of the P-wave. This VP signal is communicated to the ventricular pulse generator 16 and results in the first stimulated R-wave shown in the figure. The generation of this ventricular stimulus starts the upper rate limit timer which times out a programmed value of 500 milliseconds. The V—A time interval measurement timer 40 also starts at this time. Approximately 400 milliseconds after the first P-wave, another P-wave is generated by the atrium which is detected by the atrial sense amplifier generating the atrial sense signal shown in the figure. This P-wave initiates the operation of the A—V delay timer which again times out a 150 millisecond time interval. At the expiration of this time period the AVT generates a ventricular pace signal. However, since the generation of this signal falls within the upper rate limit time period of 500 milliseconds, the event is latched or stored within the URT 60. Therefore no ventricular pacing stimulus is generated by the ventricular pulse generator during the upper rate time period. This may be achieved by the application of an inhibit signal produced by the URT 60, and communicated to the ventricular pulse generator 16 through terminal 64. At the expiration of the upper rate limit time period of 500 milliseconds, the URT 60 generates a ventricular pace event which results in the second stimulated R-wave in the schematic EKG trace. Note that the operation of the upper rate limit timer has resulted in the extension of the A—V delay time shown on the EKG trace.

In a similar fashion, the third P-wave shown in the EKG trace is detected by the atrial sense amplifier which ultimately results in the third stimulated R-wave shown in the EKG trace. The gradual prolongation of the A—V delay time observed with this pacemaker ensures that eventually an atrial event occurs during the atrial refractory period of the pacemaker and therefore will not result in a ventricular stimulation. In this fashion, an occasional atrial beat is ignored at high rates thus stabilizing the ventricular stimulation rate at the upper rate limit. In Figure 2, the seven detected atrial P-waves have resulted in five stimulated ventricular events. This stimulation regime is called pseudo-Wenkebach pacing. It is important to note in this sequence that the measured ventricular-atrial time period has progressively decreased due to the interaction of the upper rate timer with the detected P-waves. In this example, the V—A time period measurements are not invariant.

In summary, the functional elements previously described cooperate to form an atrial synchronized pacemaker which tracks naturally occurring atrial activity up to an upper rate limit defined by the upper rate limit time period set by the URT 60.

The atrial refractory timer, ventricular-atrial measurement timer, and atrial refractory control circuit cooperate to determine whether the pacemaker is participating in or mediating a tachycardia. As described in connection with Figure 2, the pacer is not mediating the tachycardia, and the atrial refractory period is not altered.

Figure 3 shows the interaction of the pacemaker of Figure 1 with the heart in the presence of retrograde activated P-waves. In the EKG trace, the inverted P-waves indicate retrograde activated atrial activity. These P-waves result from electrical activity conducted from the ventricle to the atrium which results in an atrial depolarization. Studies have shown that such retrograde P-waves occur on the order of 160 to 330 milliseconds after ventricular stimulation. In the diagram of Figure 3, the retrograde P-waves are shown occurring approximately 200 milliseconds after the ventricular stimulating pulse.

In the figure, the first sinus P-wave is detected by the atrial sense amplifier 14 as indicated by the atrial sense spike under the P-wave in the diagram. This atrial sensed event starts the atrial refractory timer 30 as well as the A—V delay timer 20. After the expiration of the programmed A—V time interval shown as 150 milliseconds on the diagram a ventricular pace signal is generated by the AVT 20 which results in the application of a ventricular stimulating pulse to the heart. This pacing pulse is shown as a pacer artifact on the EKG line of the diagram and as a mark on the VP line of the diagram. The generation of this ventricular pace signal by the AVT 20 initiates both the upper rate timer 60 and the V—A measurement timer 40 as shown on the diagram.

The atrial refractory period ends 155 milliseconds after the ventricular stimulus. Shortly thereafter, the retrograde activated P-wave occurs shown by the inverted P-wave labeled RP on the EKG. This retro-P-wave is detected by the sense amplifier as indicated by the slash on the atrial sense line of the diagram and once again initiates the atrial refractory timer 30 as well as the A—V delay timer 20. Once again, the A—V delay timer times out a programmed time interval and generates a ventricular pace signal at the end of the time interval. However, since the upper rate limit timer has not yet timed out, this ventricular pace event is stored within the URT 60 and does not result in the generation of a ventricular pacing event.

At the expiration of the programmable upper rate limit time period the URT 60 generates a ventricular pace signal which does generate a ventricular stimulation as shown by the second R-wave complex on the EKG line of Figure 3.

As shown in the diagram, approximately 200 milliseconds after this ventricular stimulation event occurs, there is a retrograde activated P-wave which once again is sensed by the pacemaker reinitiating the process. This sensed P-wave also results in a ventricular stimulating pulse at the expiration of the upper rate limit time interval. In this fashion, the first four retrograde conducted P-waves shown on the EKG trace of Figure 3 result in ventricular stimulation at the upper rate limit of the pacemaker.

Note, however, that unlike Figure 2, the V—A time interval measurement generated by VAM 40 remains constant and varies only with the actual retrograde conduction time of the heart. It is the relative uniformity of the V—A time interval measurements which distinguish retrograde P-wave activity from the sinus tachycardia shown in Figure 2.

The preferred detection procedure for detecting the retrograde conduction includes the measurement of at least four sequential V—A time interval measurements and forming a running average based upon these mesaurements. Each of the four measurements is then compared with the running average to determine the variance in the data. If the successive time interval measurements are equal within approximately 15% of the average value, then it is likely that the pacemaker is participating in a tachycardia. In operation, logic within the atrial refractory control circuit 50 will perform these calculations and generate a control signal delivered to the atrial refractory timer 30 through terminal 38 to extend the atrial refractory period by an amount which is sufficient to prevent synchronization on the next occurring retrograde activated P-wave. This feature is shown in Figure 3 by the 50 millisecond extension of the atrial refractory period shown in the figure. This retrograde P-wave may be detected by the sense amplifier as before, however, since the detection falls within the atrial refractory period, the A—V delay timer is not initiated and there is no ventricular stimulation provided to the heart in response to the retrograde activated P-wave. In one embodiment of the invention, the atrial refractory time interval may be extended incrementally until it reaches a maximum value or until the pacemaker mediated tachycardia is interrupted. Note that in the cycle in which the retro-P-wave is blocked, the V—A time interval measurement extends appreciably; therefore, the pacer will not make any further extensions to the atrial refractory period.

Since it is desirable to have as short an atrial refractory period as possible to permit tracking of the atrial rate to high rates, it would be desirable to make the incremental extensions to the atrial refractory period relatively small, on the order of 10 milliseconds. However, since at least four cardiac cycles are required to determine whether a pacemaker mediated tachycardia is occurring, the time period required to extend the atrial refractory period may be quite long and therefore pose a risk to the patient.

In a second embodiment, the V—A time interval average value calculated by the pacemaker, increased by a fixed percentage or a fixed amount, on the order of 15% or 30 milliseconds, may be used to provide an abrupt increase in the atrial refractory period thus interrupting the tachycardia promptly after it is first detected.

In either embodiment, the atrial refractory period extending from the application of a ventricular pacing event to the heart is incremented to or set to a value which prevents the retrograde conducted P-wave from initiating an A—V delay time interval.

An additional modification to the procedure may be required to distinguish between retrograde P-waves and a sinus tachycardia at a rate just above the rate limit of the pacemaker. Returning to Figure 2, note that the V—A time interval measurement decreased as the pacemaker interacted with the heart at the atrial rate. This decrease will be monotonic for a sinus tachycardia. However, the rate at which the V—A time interval measurements decrease may be quite slow if the atrial rate is just above the rate set by the upper rate limit timer. As a consequence, four or more beats may be invariant within 15% of their ensemble average and thus be indistinguishable from retrograde P-wave activity under the procedure presented. It is anticipated, however, that the sequence of V—A time interval measurements associated with the retrograde P-wave activity will not decrease monotonically, but on the contrary will be distributed at random or in a monotonically increasing order.

This aspect may be incorporated in the detection procedure as shown in connection with Figure 4, wherein two hypothetical strings of V—A time interval measurements are compared. In Figure 4a the time interval measurements shown as M1—M4 result in an ensemble average of 233.75 milliseconds. Each of these values lies within a maximum and a minimum based upon a 15% variation around the average value. However, the measurements occur in a scattered order. As a result, this group of V—A measurements would result in the extension of the atrial refractory period.

In contrast, the same time interval measurements occurring in the sequence shown in Figure 4b will not result in the extension of the atrial refractory period. In this instance, the time interval measurements form a monotonically decreasing function which is more likely to be the result of a sinus tachycardia at the programmed upper rate limit than retrograde P-wave activity. It appears that the pathways associated with retrograde activation of atrial activity fatigue with the tachycardia and the conduction time becomes longer, therefore, the detection procedure will detect scattered data or monotonically increasing data and interpret this as originating from retrograde P-wave activity.

Although the present invention has been described within the context of a VAT pacemaker for simplicity, it should be clear that the same technique can be applied to a dual pace, dual sense pacemaker as shown in Figure 5.

The pacer of Figure 5 can operate in a variety of modes including the VAT mode described in connection with Figure 1. This pacer may also operate in the VDD or ASVIP mode as well as DDD modes.

In the VDD mode the pacer tracks the spontaneous atrial rate detected by ASA 14 from a lower rate to an upper rate. The lower rate is set by the low rate timer 70 and the upper rate is set by the upper rate limit timer 60.

The lower rate timer 70 is started by a ventricular event i.e. VS signal or VP signal applied to terminal 76 or 74 respectively, and times out a programmable escape interval. At the expiration of this interval the LRT 70 generates a ventricular stimulus. In this fashion, the LRT 70 defines the V—V interval of the pacer in the VDD mode.

The upper rate timer 60 is also started by a ventricular event applied to URT 60 through terminals 66 or 62. The URT times out a programmable time period called the upper rate limit interval. During this time period the URT 60 applies an inhibit signal through terminal 64 to the ventricular pulse generator 16 to prevent the generation of a ventricular stimulus during the upper rate time interval. If a VP signal is generated during the upper rate limit interval a latch within the URT 60 stores this information and the URT 60 will generate a VP signal at the expiration of the upper rate limit interval. This feature results in the pseudo-Wenkebach behavior described more fully in connection with Figure 2.

In the VDD mode the ventricular sense amplifier 17 is utilized to detect electrical activity within the ventricle. If a spontaneous atrial beat is followed quickly by a normally conducted ventricular beat, then the VSA 17 will detect this event and generate a VS signal which will reset the LRT 70 as well as the AVT 20. This VS signal will also start the ventricular refractory timer 80.

The ventricular refractory timer times out a refractory interval which renders the pacer insensitive to any electrical signals in the ventricle. During this interval the VRT 80 generates an inhibit signal applied to the VSA 17 through terminal 86, which prevents the generation of a VS signal during the refractory period.

In the VDD mode the retrograde conduction detection circuitry shown in Figure 5 operates as described with reference to Figure 3.

When the pacemaker of Figure 5 is operating in the DDD mode, both the atrial and ventricular pulse generators 15, 16 and the atrial and ventricular sense amplifiers, 14, 17 are activated. The sense amplifiers permit the pacemaker to monitor the naturally occurring activity of the heart and to conform the stimulation regime of the pacemaker to the requirements of the patient.

If there is no spontaneous electrical activity in either the atrium or the ventricle, the pacemaker will sequentially pace the atrium and ventricle at a rate determined by the LRT 70. In operation, the LRT V—V time interval is initiated by a ventricular event. If no additional activity is sensed by the pacemaker the LRT will generate an AP signal delivered through terminal 72 to the APG 15 which results in an atrial stimulus. After a short A—V delay period, the LRT will then issue a VP signal through terminal 74 which will generate a ventricular stimulus through the ventricular pulse generator 16. This sequential A—V pacing will occur at the programmed rate associated with the lower rate timer.

If the heart is generating a spontaneous atrial rhythm which ranges between the rate set by the LRT 70 and the upper rate set by the URT 60 then the pacemaker will respond in the atrial synchronous ventricular inhibited or VDD mode as previously described.

It is clear from the foregoing description that numerous modifications may be made, without departing from the scope of the invention.

## Claims

1. A pacer for delivering stimulation pulses to the ventricle of a patient's heart in response to detected depolarizations of the atria of the patient's heart, comprising:

an atrial terminal means (10) for coupling said pacer to the atrium of the patient's heart;

a ventricular terminal means (12) for coupling said pacer to a ventricle of the patient's heart;

atrial sense amplifier means (14) coupled to said atrial terminal means for generating an atrial sense signal in response to the detection of electrical activity in the atria;

A—V delay timer means (20) coupled to said atrial sense amplifier means for generating an A—V delay period for generating a ventricular pace signal upon the expiration of said A—V delay period, said A—V delay period being initiated by an atrial sense signal;

ventricular pulse generator means (16) coupled to said ventricular terminal means and said A—V delay timer means for producing a stimulation pulse in response to said ventricular pace signal;

an atrial refractory timer means (30) arranged to prevent the initiation of said A—V delay timer during an atrial refractory period; characterised by

detection means responsive to said atrial sense signal for detecting retrograde activated P-waves, said detection means comprising a V—A time measuring means (40) arranged to measure the time between the occurrence of a ventricular stimulating pulse and generation of an atrial sense signal and being coupled to said atrial refractory timer means for selecting said atrial refractory period to prevent retrograde activated P-waves from initiating a ventricular stimulation pulse.

2. The pacer of Claim 1 wherein said pacer further comprises:

a lower rate limit timer means coupled to said ventricular pulse generator means for defining a lower rate escape interval and for producing a ventricular pace signal upon the expiration of said lower rate escape interval, said lower rate escape timer means being initiated by the occurrence of a ventricular stimulating pulse.

3. A pacer as claimed in Claim 1 or 2 wherein the detection means is arranged to extend the atrial refractory period beyond the time after a ventricular stimulation pulse at which a retrograde P-wave occurs.

4. A pacer as claimed in Claim 1, 2 or 3 wherein the detection means is arranged to determine the running average of a plurality of V—A time measurements and to detect retrograde P-waves on the basis of the relation of individual time measurements to the running average.

5. A pacer as claimed in any preceding claim wherein the detection means is arranged to determine that retrograde P-waves are present only if successive V—A times are not monotonically decreasing.

6. A pacer as claimed in Claim 1 further comprising:

an upper rate limit timer means (60) coupled to said ventricular pulse generator means (16) for defining an upper rate limit interval for preventing the generation of a ventricular stimulating pulse during said upper rate limit interval, and for generating a ventricular pace signal at the expiration of said upper rate limit interval if a ventricular pace signal is generated during such upper rate limit period, said upper rate limit timer being initiated by the delivery of a ventricular stimulation pulse;

said atrial refractory timer means (30) being responsively coupled to said A—V delay timer means (20) for preventing the initiation of said A—V delay timer until the expiration of an atrial refractory period, said atrial refractory period extending for a first predetermined time period after the occurrence of a ventricular stimulation pulse;

said V—A time measuring means (40) being responsively coupled to said A—V delay timer means (20) and said upper rate limit timer for measuring V—A time intervals, said V—A time interval extending from the occurrence of a ventricular stimulation pulse to the subsequent initiation of said A—V delay timer means; and including

atrial refractory control means (50) coupled to said V—A measurement timer means (40) and coupled to said atrial refractory time means (30) for extending said first predetermined time period when said V—A time intervals are both short and invariant.

7. The pacer of Claim 6 further comprising:

a lower rate limit timer means coupled to said ventricular pulse generator means (16) for defining a lower rate V—V escape interval and for producing a ventricular pace signal upon the expiration of said lower rate escape interval, said lower rate escape timer means being initiated by the occurrence of a ventricular stimulating pulse.

8. A pacer for delivering stimulating pulses to the ventricle and atria of a patient's heart in response to the presence or absence of the detection of electrical activity in the atria and ventricles of the patient's heart, comprising:

atrial terminal means (10) for coupling said pacer to the atrium of the patient's heart;

ventricular terminal means (12) for coupling said pacer to a ventricle of the patient's heart;

atrial sense amplifier means (14) coupled to said atrial terminal means (10) for generating an atrial sense signal in response to the detection of electrical activity in the atria;

ventricular sense amplifier means (17) coupled to said ventricular terminal means (12) for generating a ventricular sense signal in response to the detection of electrical activity in the ventricle;

A—V delay timer means (20) responsively coupled to said atrial sense amplifier means (14) and said ventricular sense amplifier means (17) for generating an A—V delay period, said A—V delay period being initiated by an atrial sense signal and said A—V delay timer (20) being reset by said ventricular sense signal, and for generating a ventricular pace signal upon the expiration of said A—V delay period;

ventricular pulse generator means (16) coupled to said ventricular terminal means (12) for generating a ventricular stimulation signal in response to a ventricular pace signal;

an atrial pulse generator means (15) coupled to said atrial terminal means (10) for generating an atrial stimulation signal in response to an atrial pace signal;

lower rate timer means (70) coupled to said ventricular pulse generator means (16) and said atrial pulse generator means (5) and said ventricular sense amplifier means (17) for defining lower V—V and V—A rate escape intervals and for producing an atrial pace signal at the expiration of said V—A time interval and for producing a ventricular pace signal at the expiration of said V—V time interval, said lower rate timer (70) being initiated by said ventricular sense signal or said ventricular pace signal;

an atrial refractory timer means (30) responsively coupled to said A—V delay timer means (20), and to said atrial sense amplifier means (14), and to said ventricular pulse generator (16) for preventing the initiation of said A—V delay timer until the expiration of an atrial refractory period, said atrial refractory period extending for a first predetermined time period after the occurrence of a ventricular stimulation pulse;

a ventricular refractory timer means (80) coupled to said lower rate timer means (70) and said ventricular sense amplifier means (17) for defining a ventricular refractory period for preventing the initiation of said lower rate timer means (70) during said ventricular refractory period, said ventricular refractory period being initiated on the occurrence of a ventricular stimulation pulse, and said ventricular refractory period terminating a first predetermined time after initiation;

an upper rate limit timer means (60) coupled to said ventricular pulse generator means (16) for defining an upper rate limit interval for preventing the generation of a ventricular stimulating pulse during said upper rate limit interval, and for generating a ventricular pace signal at the expiration of said upper rate limit interval if a ventricular pace signal is generated during such upper rate limit period, said upper rate limit timer being initiated by the occurrence of a ventricular event, characterised by;

a V—A measurement timer means (40) responsively coupled to said A—V timer (20) and to said ventricular pulse generator (16) for measuring the time period from the occurrence of a ventricular stimulation pulse to the subsequent initiation of said A—V delay timer (20); and

atrial refractory control means (50) coupled to said V—A measurement timer means (40) and coupled to said atrial refractory timer means (30) for extending said first predetermined time period when said V—A time periods are both short and invariant.

9. A pacer as claimed in any of Claims 6 to 8 wherein:

said atrial refractory control means (50) utilizes the occurrence of two or more ventricular stimulating signals at said upper rate limit to ascertain that the V—A time period measurements meet the shortness criteria;

said atrial refractory control means (50) averages two or more successive V—A time period measurements to generate an average V—A time period measurement;

said atrial refractory control means (50) compares each of said V—A time period measurements with said average to generate successive difference measurements; and

said atrial refractory control means (50) extends said atrial refractory period if said differences are less than a preestablished difference and said successive ventricular pace signals are at said upper rate limit.

**Patentansprüche**

1. Schrittmacher zum Anleifern von Reizimpulsen an den Ventrikel des Herzens eines Patienten aufgrund von erfaßten Verhofdepolarisationen des Herzens des Patienten, mit:

einer atrialen Anschlußanordnung (10) zum Ankoppeln des Schrittmachers an den Vorhof des Herzens des Patienten;

einer ventrikulären Anschlußanordnung (12) zum Ankoppeln des Schrittmachers an einen Ventrikel des Herzens des Patienten;

einer an die atriale Anschlußanordnung angekoppelten atrialen Wahrnehmungsverstärkeranordnung (14) zum Erzeugen eines atrialen Wahrnehmungssignals aufgrund der Erfassung von elektrischer Aktivität in den Vorhöfen;

einer mit der atrielen Wahrnemungsverstärkeranordnung gekoppelten A—V-Verzögerungszeitgeberanordnung (20) zum Erzeugen einer A—V-Verzögerungsdauer zwecks Erzeugung eines ventrikulären Schrittmachersignals nach Ablauf der von einem atrialen Wahrnehmungssignal initiierten A—V-Verzögerungsdauer;

einer an die ventrikuläre Anschlußanordnung und an die A—V-Verzögerungszeitgeberanordnung angekoppelten ventrikulären Impulsgeneratoranordnung (16) zum Erzeugen eines Reizimpulses aufgrund des ventrikulären Schrittmachersignals; und

einer atrialen Refraktärzeitgeberanordnung (30) zum Verhindern des Auslösens der atrialen A—V-Verzögerungszeitgeberanordnung während einer atrialen Refraktärdauer, gekennzeichnet, durch eine auf das atriale Wahrnehmungssignal ansprechende Erfassungsanordnung zum Erfassen von retrograd aktivierten P-Wellen, wobei die Erfassungsanordnung eine V—A-Zeitmeßanordnung (40) aufweist, die zum Messen der Zeitspanne zwischen dem Auftreten eines ventrikulären Reizimpulses und dem Erzeugen eines atrialen Erfassungssignals ausgelegt und mit der atrialen Refraktärzeitgeberanordnung zwecks Auswahl der atrialen Refraktärdauer gekoppelt ist, um retrograd aktivierte P-Wellen am Auslösen eines ventrikulären Reizimpulses zu hindern.

2. Schrittmacher nach Anspruch 1 ferner versehen mit:

einer mit der ventrikulären Impulsgeneratoranordnung gekoppelten Zeitgeberanordnung für einen unteren Folgefrequenzgrenzwert zur Vorgabe eines unteren Folgefrequenz-Escapeintervalls und zum Erzeugen eines ventrikulären Schrittmachersignals nach dem Verstreichen des unteren Folgefrequenz-Escapeintervalls, wobei die Zeitgeberanordnung für das untere Folgefrequenz-Escapeintervall durch das Auftreten eines ventrikulären Reizimpulses ausgelöst wird.

3. Schrittmacher nach Anspruch 1 oder 2, wobei die Erfassungsanordnung zum Verlängern der atrialen Refraktärdauer über die Zeit nach einem ventrikulären Reizimpuls, bei welcher eine retrograde P-Welle auftritt, ausgelegt ist.

4. Schrittmacher nach Anspruch 1, 2 oder 3, wobei die Erfassungsanordnung für das Bestimmen des laufenden Mittelwertes einer Mehrzahl von V—A-Zeitmessungen und für das Erfassen von retrograden P-Wellen auf der Basis der Relation von einzelnen Zeitmessungen zu dem laufenden Mittelwert ausgelegt ist.

5. Schrittmacher nach einem der vorhergehenden Ansprüche, wmbei die Erfassungsanordnung derart ausgelegt ist, daß sie das Vorhandensein von retrograden P-Wellen nur bestimmt, falls aufeinanderfolgende V—A-Dauern nicht monoton abnehmen.

6. Schrittmacher nach Anspruch 1 ferner versehen mit:

einer mit der ventrikulären Impulsgeneratoranordnung (16) gekoppelten Zeitgeberanordnung (60) für einen oberen Folgefrequenzgrenzwert zur Vorgabe eines oberen Folgefrequenzgrenzwertintervalls zum Verhindern der Erzeugung eines ventrikulären Reizimpulses während dieses oberen Folgefrequenzgrenzwertintervalls und zum Erzeugen eines ventrikulären Schrittmachersignals bei Ablauf des oberen Folgefrequenzgrenzwertintervalls, falls während dieser oberen Ratengrenzwertdauer ein ventrikuläres Schrittmachersignal erzeugt wird, wobei die Zeitgeberanordnung für den oberen Folgefrequenzgrenzwert durch die Abgabe eines ventrikulären Reizimpulses ausgelöst wird;

wobei die atriale Refraktärzeitgeberanordnung (30) mit der A—V-Verzögerungszeitgeberanordnung (20) für eine Verhinderung des Auslösens der A—V-Verzögerungszeitgeberanordnung bis zum Ablauf einer atrialen Refraktärdauer gekoppelt ist, die für eine erste vorbestimmte Zeitdauer nach dem Auftreten eines ventrikulären Reizimpulses andauert;

wobei die V—A-Zeitmeßanordnung (40) mit der A—V-Verzögerungszeitgeberanordnung (20) und mit der Zeitgeberanordnung für einen oberen Folgefrequenzgrenzwert zwecks Messung von V—A-Zeitintervallen gekoppelt ist, die von dem Auftreten eines ventrikulären Reizimpulses bis zu dem nachfolgenden Auslösen der A—V-Verzögerungszeitgeberanordnung andauern; sowie mit

einer atrialen Refraktärsteueranordnung (50), die mit der V—A-Zeitmeßanordnung (40) und mit der atrialen Refraktärzeitgeberanordnung (30) gekoppelt ist, um die erste vorbestimmte Zeitdauer zu verlängern, wenn die V—A-Zeitintervalle sowohl kurz als auch invariant sind.

7. Schrittmacher nach Anspruch 6 ferner versehen mit:

einer mit der ventrikulären Impulsgeneratoranordnung (16) gekoppelte Zeigeberanordnung für einen unteren Folgefrequenzgrenzwert zur Vorgabe eines unteren Folgefrequenz-V—V-Escapeintervalls und zum Erzeugen eines ventrikulären Schrittmachersignals nach dem Verstreichen des unteren Folgefrequenz-Escapeintervalls, wobei die Zeitgeberanordnung für das untere Folgefrequenz-Escapeintervall durch das Auftreten eines ventrikulären Reizimpulses ausgelöst wird.

8. Schrittmacher zum Anliefern von Reizimpulsen an den Ventrikel und die Vorhöfe des Herzens eines Patienten aufgrund des Vorliegens oder Nichtvorliegens der Erfassung von elektrischer Aktivität in den Vorhöfen und den Ventrikeln des Herzens des Patienten, mit:

einer atrialen Anschlußanordnung (10) zum Ankoppeln des Schrittmachers an den Vorhof des Herzens des Patienten;

einer ventrikulären Anschlußanordnung (12) zum Ankoppeln des Schrittmachers an einen Ventrikel des Herzens des Patienten;

einer an die atriale Anschlußanordnung (10) angekoppelten atrialen Wahrnehmungsverstärkeranordnung (14) zum Erzeugen eines atrialen Wahrnehmungssignals aufgrund der Erfassung von elektrischer Aktivität in den Vorhöfen;

einer an die ventrikuläre Anschlußanordnung (12) angekoppelten ventrikulären Wahrnehmungsverstärkeranordnung (17) zum Erzeugen eines ventrikulären Wahrnehmungssignals aufgrund der Erfassung von elektrischer Aktivität in dem·Ventrikel;

einer mit der atrialen Wahrnehmungsverstärkeranordnung (14) und der ventrikulären Wahrnehmungsverstärkeranordnung (17) gekoppelten A—V-Verzögerungszeitgeberanordnung (20) zum Erzeugen einer von einem atrialen Erfassungssignal ausgelösten A—V-Verzögerungsdauer und zum Erzeugen eines ventrikulären Schrittmachersignals nach Ablauf der A—V-Verzögerungsdauer, wobei die A—V-Verzögerungszeitgeberanordnung (20) mittels des ventrikulären Wahrnehmmungssignals zurückgestellt wird;

einer an die ventrikuläre Anschlußanordnung (12) angekoppelten ventrikulären Impulsgeneratoranordnung (16) zum Erzeugen eines ventrikulären Reizsignals aufgrund eines ventrikulären Schrittmachersignals;

einer an die atriale Anschlußanordnung (10) angekoppelten atrialen Impulsgeneratoranordnung (15) zum Erzeugen eines atrialen Reizsignals aufgrund eines atrialen Schrittmachersignals;

einer an die ventrikuläre Impulsgeneratoranordnung (16) und die atriale Impulsgeneratoranordnung (15) sowie die ventrikuläre Wahrnehmungsverstärkeranordnung (17) angekoppelte untere Folgefrequenz-Zeitgeberanordnung (70) zur Vorgabe von unteren V—V- und V—A-Folgefrequenz-Escapeintervallen und zum Erzeugen eines atrialen Schrittmachersignals beim Ablauf des V—A-Zeitintervalls sowie zum Erzeugen eines ventrikulären Schrittmachersignals beim Ablauf des V—V-Zeitintervalls, wobei die untere Folgefrequenz-Zeitgeberanordnung (70) durch das ventrikuläre Wahrnehmungssignal oder das ventrikuläre Schrittmachersignals ausgelöst wird;

einer mit der A—V-Verzögerungszeitgeberanordnung (20) und der atrialen Wahrnehmungsverstärkeranordnung (14) sowie dem ventrikulären Impulsgenerator (16) gekoppelten atrialen Refraktärzeitgeberanordnung (30) zum Verhindern des Auslösens der A—V-Verzögerungszeitgeberanordnung bis zum Ablauf einer atrialen Refraktärdauer, die für eine erste vorbestimmte Zeitdauer nach dem Auftreten eines ventrikulären Reizimpulses andauert;

einer mit der unteren Folgefrequenz-Zeitgeberanordnung (70) und der ventrikulären Wahrnehmungsverstärkeranordnung (17) gekoppelten ventrikulären Refraktärzeitgeberanordnung (80) zur Vorgabe einer ventrikulären Refraktärdauer für ein Verhindern des Auslösens der unteren Folgefrequenz-Zeitgeberanordnung (70) während der ventrikulären Refraktärdauer, die beim Auftreten eines ventrikulären Reizimpulses ausgelöst wird und die nach einer ersten vorbestimmten Zeitspanne nach dem Auslösen endet;

einer mit der ventrikulären Impulsgeneratoranordnung (16) gekoppelten oberen Grenzfolgefrequenz-Zeitgeberanordnung (60) zur Vorgabe eines oberen Folgefrequenz-Grenzwertintervalls zum Verhindern der Erzeugung eines ventrikulären Reizimpulses während des oberen Folgefrequenzgrenzwertintervalls und zum Erzeugen eines ventrikulären Schrittmachersignals bei Ablauf des oberen Folgefrequenzgrenzwertintervalls, falls während der oberen Folgefrequenzgrenzwertdauer ein ventrikuläres Schrittmachersignal erzeugt wird, wobei die obere Grenzfolgefrequenz-Zeitgeberanordnung durch das Auftreten eines ventrikulären Ereignisses ausgelöst wird; gekennzeichnet durch:

an die A—V-Zeitgeberanordnung (20) und den ventrikulären Impulsgenerator (16) angekoppelte V—A-Messungs-Zeitgeberanordnung (40) zum Messen der Zeitdauer von dem Auftreten eines ventrikulären Reizimpulses bis zu dem nachfolgenden Auslösen der A—V-Verzögerungszeitgeberanordnung (20); und

eine mit der V—A-Messungs-Zeitgeberanordnung (40) und der atrialen Refraktärzeitgeberanordnung (30) gekoppelten atrialen Refraktärsteueranordnung (50) zum Verlängern der ersten vorbestimmten Zeitdauer, wenn die V—A-Zeitdauern sowohl kurz als auch invariant sind.

9. Schrittmacher nach einem der Ansprüche 6 bis 8, wobei:

die atriale Refraktärsteueranordnung (50) das Auftreten von zwei oder mehr ventrikulären Reizsignalen mit dem oberen Folgefrequenzgrenzwert zu der Feststellung nutzt, daß die V—A-Zeitdauermessungen die Kürzekriterien erfüllen;

die atriale Refraktärsteueranordnung (50) für eine mittlere V—A-Zeitdauermessung zwei oder mehr aufeinanderfolgende V—A-Zeitdauermessungen mittelt;

die atriale Refraktärsteueranordnung (50) jeder der V—A-Zeitdauermessungen mit dem Mittelwert für aufeinanderfolgende Differenzmessungen vergleicht; und

die atriale Refraktärsteueranordnung (50) die atriale Refraktärdauer verlängert, wenn die Differenzen kleiner als eine vorbestimmte Differenz sind und die aufeinanderfolgenden ventrikulären Schrittmachersignale mit dem oberen Folgefrequenzgrenzwert auftreten.

**Revendications**

1. Stimulateur destiné à délivrer des impulsions de stimulation au ventricule du coeur d'un malade en réponse à des dépolarisations détectées des oreillettes du coeur du malade, comportant:

un dispositif de connexion auriculaire (10) destiné à coupler ledit stimulateur avec l'oreillette du coeur du malade;

un dispositif de connexion ventriculaire (12) destiné à coupler ledit stimulateur avec un ventricule du coeur du malade;

un amplificateur de détection auriculaire (14) couplé avec ledit dispositif de connexion auriculaire pour produire un signal de détection auriculaire en réponse à la détection d'un activité électrique dans les oreillettes;

un temporisateur de retard A—V (20) couplé avec ledit amplificateur de détection auriculaire pour produire une période de retard A—V afin d'émettre un signal de stimulation ventriculaire à l'expiration de ladite période de retard A—V, ladite période de retard A—V étant déclenchée par un signal de détection auriculaire;

un générateur d'impulsions ventriculaires (16) couplé avec ledit dispositif de connexion ventriculaire et ledit temporisateur de retard A—V pour produire une impulsion en réponse audit signal de stimulation ventriculaire;

un temporisateur réfractaire auriculaire (30) agencé pour interdire le déclenchement dudit temporisateur de retard A—V pendant une période auriculaire réfractaire; caractérisé par:

un dispositif de détection réagissant audit signal de détection auriculaire en détectant des ondes P activées rètrogrades, ledit dispositif de détection comportant un dispositif de mesure de temps V—A (40) agencé pour mesurer le temps entre l'apparition d'une impulsion de stimulation ventriculaire et la production d'un signal de détection auriculaire et étant couplé avec ledit temporisateur réfractaire auriculaire pour sélectionner ladite période réfractaire auriculaire afin d'interdire que des ondes P activées rétrogrades déclenchent une impulsion de stimulation ventriculaire.

2. Stimulateur selon la revendication 1, dans lequel ledit stimulateur comporte en outre;

un temporisateur de limite de fréquence inférieure couplé avec ledit générateur d'impulsions ventriculaires pour définir un intervalle d'échappement de fréquence inférieure et pour produire un signal de stimulation ventriculaire à l'expiration dudit intervalle d'échappement de fréquence inférieure, ledit temporisateur d'échappement de fréquence inférieure étant déclenché par l'apparition d'une impulsion de stimulation ventriculaire.

3. Stimulateur selon la revendication 1 ou 2, dans lequel le dispositif de détection est agencé pour prolonger la période réfractaire auriculaire au-delà de l'instant après une impulsion de stimulation ventriculaire à laquelle apparaît une onde P rétrograde.

4. Stimulateur selon la revendication 1, 2 ou 3, dans lequel le dispositif de détection est agencé pour déterminer la moyenne en cours de plusieurs mesures de temps V—A et pour détecter des ondes P rétrogrades sur la base de la relation des mesures de temps individualles et de la moyenne en cours.

5. Stimulateur selon l'une quelconque des revendications précédentes, dans lequel le dispositif de détection est agencè pour déterminer, que des ondes P rétrogrades sont présentes seulement si des temps V—A successifs ne décroissent pas de façon monotone.

6. Stimulateur selon la revendication 1, comportant en outre:

un temporisateur de limite de fréquence supérieure (60) couplé avec ledit générateur d'impulsions ventriculaires (16) pour définir un intervalle limite de fréquence supérieure afin d'interdire la production d'une impulsion de stimulation ventriculaire pendant ledit intervalle limite de fréquence supérieure et pour produire un signal de stimulation ventriculaire à l'expiration dudit intervalle limite de fréquence supérieure si un signal de stimulation ventriculaire est produit pendant cette période limite de fréquence supérieure, ledit temporisateur de limite de fréquence supérieure étant déclenché par la délivrance d'une impulsion de stimulation ventriculaire;

ledit temporisateur réfractaire auriculaire (30) étant couplé de manière à réagir audit temporisateur de retard A—V (20) pour interdire le déclenchement dudit temporisateur de retard A—V jusqu'à l'expiration d'une période réfractaire auriculaire, ladite période réfractaire auriculaire s'étendant pendant une première période de temps prédéterminée après l'apparition d'une impulsion de stimulation ventriculaire;

ledit dispositif de mesure de temps V—A (40) étant couplé pour réagir audit temporisateur de retard A—V (20) et audit temporisateur de limite de fréquence supérieure pour mesurer des intervalles de temps V—A, ledit intervalle de temps V—A s'étendant depuis l'apparition d'une impulsion de stimulation ventriculaire jusqu'au déclenchement suivant dudit temporisateur de retard A—V; et comportant

un dispositif de commande réfractaire auriculaire (50) couplé avec ledit temporisateur de mesure V—A (40) et couplé avec ledit temporisateur réfractaire auriculaire (30) pour prolonger ladite période de temps prédéterminée quand lesdits intervalles de temps V—A sont à la fois courts et invariables.

7. Stimulateur selon la revendication 6, comportant en outre:

un temporisateur de limite de fréquence inférieure couplé avec ledit générateur d'impulsions ventriculaires (16) pour définir un intervalle d'échappement V—V de fréquence inférieure et pour produire un signal de stimulation ventriculaire à l'expiration dudit intervalle d'échappement de fréquence inférieure, ledit temporisateur d'échappement de fréquence inférieure étant déclenché par l'apparition d'une impulsion de stimulation ventriculaire.

8. Stimulateur destiné à délivrer des impulsions de stimulation aux ventricules et aux oreillettes du coeur d'un malade en réponse à la présence ou l'absence de la détection d'une activité électrique dans les oreillettes et les ventricules du coeur du malade, comportant:

un dispositif de connexion auriculaire (10) destiné à coupler ledit stimulateur avec l'oreillette du coeur d'un malade:

un dispositif de connexion ventriculaire (12) destiné à coupler ledit stimulateur avec un ventricule du coeur du malade;

un amplificateur de détection auriculaire (14) couplé avec ledit dispositif de connexion auriculaire (10) pour produire un signal de détection auriculaire en réponse à la détection d'une activité électrique dans les oreillettes;

un amplificateur de détection ventriculaire (17) couplé avec ledit dispositif de connexion ventriculaire (12) pour produire un signal de détection ventriculaire en réponse à la détection d'une activité électrique dans le ventricule;

un temporisateur de retard A—V (20) couplé pour réagir audit amplificateur de détection auriculaire (14) et audit amplificateur de détection ventriculaire (17) pour produire une période de retard A—V ladite période de retard A—V étant déclenchée par un signal de détection auriculaire et ledit temporisateur de retard A—V (20) étant ramené au repos par ledit signal de détection ventriculaire et pour produire un signal de stimulation ventriculaire à l'expiration de ladite période de retard A—V;

un générateur d'impulsions ventriculaires (16) couplé avec ledit dispositif de connexion ventriculaire (12) pour produire un signal de stimulation ventriculaire en réponse à un signal de stimulation ventriculaire;

un générateur d'impulsions auriculaires (15) couplé avec ledit dispositif de connexion auriculaire (10) pour produire un signal de stimulation auriculaire en réponse à un signal de stimulation auriculaire;

un temporisateur de fréquence inférieure (70) couple avec ledit générateur d'impulsions ventriculaires (16) et ledit générateur d'impulsions auriculaires (5) et ledit amplificateur de détection ventriculaire (17) pour définir des intervalles d'échappement de fréquence inférieure V—V et V—A et pour produire un signal de stimulation auriculaire à l'expiration dudit intervalle de temps V—A et pour produire un signal de stimulation ventriculaire à l'expiration dudit intervalle de temps V—V, ledit temporisateur de fréquence inférieure (70) étant déclenché par ledit signal de détection ventriculaire ou ledit signal de stimulation ventriculaire;

un temporisateur réfractaire auriculaire (30) couplé pour réagir audit temporisateur de retard A—V (20) et audit amplificateur de détection auriculaire (14) et audit générateur d'impulsions ventriculaires (16) pour interdire le déclenchement dudit temporisateur de retard A—V jusq'à l'expiration d'une période réfractaire auriculaire, ladite période réfractaire auriculaire s'étendant pendant une première période de temps prédéterminée apres l'apparition d'une impulsion de stimulation ventriculaire;

un temporisateur réfractaire ventriculaire (80) couplé avec ledit temporisateur de fréquence inférieure (70) et ledit amplificateur de détection ventriculaire (17) pour définir une période réfractaire ventriculaire afin d'interdire le déclenchement dudit temporisateur de fréquence inférieure (70) pendant ladite période réractaire auriculaire, ladite période réfractaire ventriculaire étant déclenchée à l'apparition d'une impulsion de stimulation ventriculaire et ladite période réfractaire ventriculaire se terminant à un premier instant prédéterminé après le déclenchement;

un temporisateur de limite de fréquence supérieure (60) couplé avec ledit générateur d'impulsions ventriculaires (16) pour définir un intervalle limite de fréquence supérieure afin d'interdire la production d'une impulsion de stimulation ventriculaire pendant ledit intervalle limite de fréquence supérieure et pour produire un signal de stimulation ventriculaire à l'expiration dudit intervalle limite de fréquence supérieure si un signal de stimulation ventriculaire est produit pendant cette période limit de fréquence supérieure, ledit temporisateur de limite de fréquence supérieure étant déclenché à l'apparition d'un évènement ventriculaire, caractérisé par:

un temporisateur de mesure V—A (40) couplé pour réagir audit temporisateur A—V (20) et audit générateur d'impulsions ventriculaires (16) pour mesurer la période de temps depuis l'apparition d'une impulsion de stimulation ventriculaire jusqu'au déclenchement suivant dudit temporisateur de retard A—V (20); et

un dispositif de commande réfractaire auriculaire (50) couplé avec ledit temporisateur de mesure V—A (40) et couplé avec ledit temporisateur réfractaire auriculaire (30) pour prolonger ladite première période de temps prédéterminée quand lesdites périodes de temps V—A sont à la fois courtes et invariables.

9. Stimulateur selon l'une quelconque des revendications 6 à 8, dans lequel:

ledit dispositif de commande réfractaire auriculaire (50) utilise l'apparition de deux ou plusieurs signaux de stimulation ventriculaire à ladite limite de fréquence supérieure pour déterminer que les mesures de période de temps V—A répondent au critère de courte durée;

ledit dispositif de commande réfractaire auriculaire (50) faisant la moyenne de deux ou plusieurs mesures de période de temps V—A successives pour produire une mesure de période de temps V—A moyenne;

ledit dispositif de commande réfractaire auriculaire (50) comparant chacune desdites mesures de période de temps V—A avec ladite moyenne pour produire des mesures de différences successives; et

ledit dispositif de commande réfractaire auriculaire (50) prolongeant ladite période réfractaire auriculaire si lesdites différences sont inférieures à une différence préétablie et lesdits signaux de stimulation ventriculaire successifs sont à ladite limite de fréquence supérieure.

Fig. 1

Fig. 4

_Fig. 2_

_Fig. 3_

Fig. 5